Europäisches Patentamt

European Patent Office

Office européen des brevets

(19)

(11) **EP 0 718 616 B1**

(12) **FASCICULE DE BREVET EUROPEEN**

(45) Date de publication et mention
de la délivrance du brevet:
**02.05.2003 Bulletin 2003/18**

(51) Int Cl.7: **G01N 15/08**, G01N 33/24

(21) Numéro de dépôt: **95402862.7**

(22) Date de dépôt: **18.12.1995**

(54) **Méthode et dispositif de mesure en continu des variations de la saturation globale d'un échantillon en fluides non miscibles incompressibles**

Verfahren und Anordnung zur kontinuierlichen Messung der Sättigungsänderungen einer Probe mit nichtmischbaren inkompressiblen Fluiden

Method and device for continuously measuring variations of saturation of a sample with non-mixable incompressible fluids

(84) Etats contractants désignés:
**DE GB IT**

(30) Priorité: **19.12.1994 FR 9415375**

(43) Date de publication de la demande:
**26.06.1996 Bulletin 1996/26**

(73) Titulaire: **INSTITUT FRANCAIS DU PETROLE**
**92500 Rueil Malmaison (FR)**

(72) Inventeur: **Lemaire, Christian**
**D-92000 Nanterre (FR)**

(56) Documents cités:
**US-A- 4 487 056      US-A- 4 543 821**
**US-A- 4 672 840      US-A- 5 086 643**
**US-A- 5 209 104      US-A- 5 299 453**

• **REVUE DE L'INSTITUT FRANCAIS DU PETROLE, vol. 45, no. 4, Juillet 1990 PARIS FR, pages 489-506, XP 000176900 XH KOCI ET AL.**

## Description

La présente invention concerne une méthode de mesure en continu des variations de la saturation globale en fluide d'un échantillon de matériau poreux soumis à un drainage ou une imbibition, suivant le principe d'une pesée en continu à volume constant, ainsi qu'un dispositif pour sa mise en oeuvre.

La méthode selon l'invention convient notamment pour l'étude d'échantillons géologiques prélevés dans des formations recelant ou susceptibles de receler des effluents pétroliers.

La connaissance que l'on peut acquérir de différents paramètres pétrophysiques des roches durant des phases de drainage ou d'imbibition, peut servir par exemple à choisir le fluide le plus apte pour déplacer les effluents pétroliers qu'elles contiennent et d'améliorer de ce fait l'efficacité des procédés de récupération assistée d'effluents dans un gisement.

Il est connu de déterminer par exemple la saturation et la mouillabilité des roches vis-à-vis de fluides tels que l'eau (sous forme de saumure) et l'huile qui peuvent y être contenus. A cet effet, on procède à des phases de drainage de la roche c'est-à-dire à un déplacement des fluides visant à diminuer la saturation en eau, suivies de phases d'imbibition, visant au contraire à augmenter sa saturation en eau (Sw). La pression capillaire en un point d'un échantillon poreux en présence d'eau et d'huile en phase continue, se définit, on le rappelle, comme la différence Pc à l'équilibre entre la pression P(huile) et la pression P(eau) de l'eau. Des dispositifs permettant de mesurer des paramètres pétrophysiques de roches sont décrits par exemple dans les demandes de brevets EP 603 040, FR 2 708 742 ou FR 2 724 460 du demandeur ou bien encore les brevets US 4 868 751, 5 069 065 ou 5 086 643.

Par le brevet US 5 086 643, on connaît une méthode et un dispositif pour faire des mesures de saturation sur un échantillon de roche poreux, par drainage d'un premier fluide saturant hors de la cellule au moyen d'une injection sous pression d'un deuxième fluide incompressible, de masse spécifique connue et non miscible avec le premier. Les fluides issus de la cellule sont envoyés dans un séparateur transparent permettant des mesures "visuelles" par un opérateur, avec des "piquages" permettant sur commande, au moyen de pompes et de débitmètres, de connaître le volume des phases séparées dans le séparateur.

La méthode selon l'invention est définie dans la revendication 1 et permet de mesurer automatiquement les variations de saturation globale d'un échantillon présentant une certaine porosité, que l'on a saturé initialement avec un premier fluide quand on injecte à travers lui un deuxième fluide incompressible de masse spécifique connue différente de celle du premier fluide et non miscible avec lui.

La méthode comporte une collecte des fluides issus de l'échantillon suite à l'injection du deuxième fluide dans un séparateur diphasique. Elle est caractérisée en ce qu'elle comporte le remplissage initial complet du séparateur de phases avec le premier fluide, la mesure en continu des variations de la masse du séparateur de phases avec évacuation progressive du premier fluide en excès, permettant une discrimination automatique des contributions des deux phases à cette variation de la masse, et le calcul des variations en fonction du temps de la saturation globale de l'échantillon à partir desdites variations de masse.

Suivant un mode de mise en oeuvre, on sature l'échantillon initialement avec de l'eau et on déplace cette eau hors de l'échantillon par une injection d'huile. Suivant un autre mode de mise en oeuvre, on peut inversement saturer l'échantillon initialement avec de l'huile et déplacer cette huile hors de l'échantillon par une injection d'eau.

Le dispositif selon l'invention est défini dans la revendication 4 et permet de mesurer en continu les variations de saturation globale d'un échantillon présentant une certaine porosité, que l'on a saturé initialement avec un premier fluide, quand on injecte à travers lui un deuxième fluide non miscible avec le premier fluide et de masse spécifique différente de celle du premier fluide. Il comporte des moyens pour injecter du deuxième fluide dans l'échantillon, un séparateur diphasique pour recueillir les fluides issus de l'échantillon suite à l'injection du deuxième fluide, et des moyens d'évacuation du premier fluide en excès. Le dispositif est caractérisé en ce qu'il comporte des moyens de pesage du séparateur diphasique, produisant des signaux indicatifs des variations de la masse du séparateur diphasique, des moyens automatiques de traitement des signaux produits par les moyens de pesage pour déterminer par discrimination les masses respectives du premier fluide et du deuxième fluide recueillis dans le séparateur diphasique, ainsi que les variations continues de saturation de l'échantillon.

Le séparateur comporte par exemple une cellule pourvue d'une entrée pour une canalisation de collecte des fluides évacués de l'échantillon, et d'une sortie pour la connexion d'une canalisation permettant l'évacuation du premier fluide en excès.

Les moyens de traitement des signaux comportent par exemple un processeur programmé pour déterminer les variations de saturation à partir des variations de la masse du séparateur et des masses spécifiques respectives du premier et du deuxième fluide.

Avec la méthode et le dispositif selon l'invention, on peut mesurer automatiquement en fonction du temps et sur de longues périodes, tout un processus de saturation progressive d'un échantillon au contact de deux fluides différents.

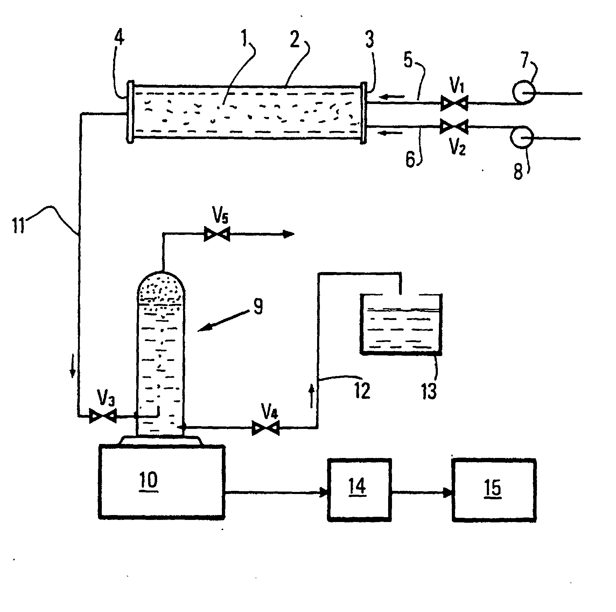

La méthode pour mesurer les variations de la saturation globale d'un échantillon selon l'invention, peut être mise en oeuvre par exemple avec le dispositif schématisé à la Fig. 1, qui va être décrit ci-après, à titre d'exemple non limitatif.

Le barreau d'échantillon 1 à tester est enrobé étroitement par exemple dans une gaine 2 en résine qui réalise l'étanchéité latérale. Aux deux extrémités de la gaine, contre les parois terminales du barreau, sont appliquées directement deux plaques d'embout ou chapeaux 3, 4. La première plaque d'embout 3 est pourvue d'alésages pour le raccordement de conduits 5, 6 communiquant par l'intermédiaire de vannes, V1, V2 avec une pompe d'injection d'eau 7 et/ou une pompe d'injection d'huile 8. Des prises de pression sélectives à membranes semi-perméables PP, telles que décrites par exemple dans la demande de brevet parallèle EN 94/15376 au nom du demandeur, peuvent être insérées également au travers de la gaîne en différents emplacements le long de l'échantillon, de façon de mesurer sélectivement les pressions des différentes phases au sein de l'échantillon et permettre par exemple le calcul de pressions capillaires.

Le dispositif comporte en outre un séparateur diphasique 9 constitué d'un récipient allongé ou cellule réalisé en verre par exemple, qui est posé sur une balance 10. La cellule 9 communique avec la plaque d'embout opposée 4 par une canalisation 11 munie d'une vanne de contrôle V3, qui débouche vers sa base. Une autre canalisation 12 munie d'une vanne de contrôle V4, fait communiquer la partie basse du séparateur 9 avec un récipient de collecte 13. Un orifice supérieur contrôlé par une autre vanne V5, permet l'échappement hors du séparateur 9, d'une phase gazeuse éventuelle. La balance10 est du type électronique. Les signaux de mesure de la masse de la cellule 9 délivrés par la balance 10 sont appliqués à une entrée d'acquisition d'un processeur 14 programmé pour effectuer le suivi des tests comme il sera indiqué ci-après, ce processeur étant connecté à une console de commande 15.

On utilise un échantillon poreux sec par exemple que l'on sature d'abord sous vide avec de la saumure. On procède ensuite à la mise en place d'une saturation irréductible en eau, cette opération consistant par exemple à injecter de l'huile avec un débit constant dans l'échantillon saturé au moyen de la pompe 8 jusqu'à atteindre un niveau minimal irréductible de saturation en eau. On remplit totalement de saumure le séparateur 9 ainsi que la canalisation 11, et on initialise la balance 10 à zéro.

On ferme la vanne V2 et, les vannes V3 et V4 étant ouvertes, on injecte de l'eau au travers de la plaque 3 dans le barreau d'échantillon 1 au moyen de la pompe 7. Un mélange constitué d'eau et d'huile déplacée par l'injection sort par la canalisation 11 et pénètre dans le séparateur 9. L'huile s'accumule dans la partie supérieure de la cellule 9, et l'eau en excès s'échappe vers le récipient de collecte 13. Globalement, la masse du séparateur 9 décroît et la balance 10. enregistre une variation négative de la masse par rapport au zéro initial.

A partir des variations négatives de la masse M(t) en fonction du temps t, enregistrées par la balance 10 et acquises par le processeur 14, celui-ci calcule en continu les variations symétriques du volume d'huile Vo et d'eau Vw dans la cellule 9 par les relations suivantes:

$$Vo = -M(t)/(Ro - Rw);$$

et

$$Vw = M(t)/(Rw - Ro),$$

(Rw - Ro) représentant la différence des masses spécifiques respectivement de l'eau et de l'huile. Le processeur 14 détermine également les variations en fonction du temps également des saturations de l'échantillon en eau et en huile, en les rapportant aux volumes des pores du barreau.

On a décrit à titre d'exemple un processus de déplacement d'eau dans un échantillon de roche par de l'huile injectée. Il est bien évident que l'on pourrait tester aussi bien dans des conditions semblables un déplacement d'huile hors de l'échantillon par de l'eau injectée en modifiant en conséquence bien évidemment la position des orifices de communication du séparateur 9 avec les canalisations 11, 12.

D'une façon plus générale la méthode décrite convient pour tout processus où l'on déplace dans un échantillon un premier fluide saturant par un deuxième fluide injecté, ces deux fluides étant immiscibles et incompressibles.

Avec l'agencement qui a été décrit, on peut suivre automatiquement le déroulement d'un processus de saturation biphasique sur de longues périodes.

## Revendications

1. Méthode pour mesurer les variations de saturation globale d'un échantillon présentant une certaine porosité, que l'on a saturé initialement avec un premier fluide quand on injecte à travers lui un deuxième fluide incompressible de masse spécifique connue différente de celle du premier fluide et non miscible avec lui, dans laquelle on recueille les fluides issus de l'échantillon suite à l'injection du deuxième fluide dans un séparateur diphasique (9), **caractérisée en ce qu'**elle comporte le remplissage initial complet du séparateur de phases avec le premier fluide, la mesure en continu des variations de la masse du séparateur de phases avec évacuation progressive du premier fluide en excès, permettant une discrimination automatique des contributions des deux phases à cette variation de la masse, et le calcul des variations en fonction du temps de la saturation globale de l'échantillon à partir desdites variations de masse.

2. Méthode selon la revendication 1, **caractérisée en**

**ce que** l'on sature l'échantillon initialement avec de l'eau et on déplace cette eau hors de l'échantillon par une injection d'huile.

3. Méthode selon la revendication 1, **caractérisée en ce que** l'on sature l'échantillon initialement avec de l'huile et on déplace cette huile hors de l'échantillon par une injection d'eau.

4. Dispositif pour mesurer en continu les variations de saturation globale d'un échantillon présentant une certaine porosité, que l'on a saturé initialement avec un premier fluide, quand on injecte à travers lui un deuxième fluide non miscible avec le premier fluide et de masse spécifique différente de celle du premier fluide, comportant des moyens (3, 5-8) pour injecter du deuxième fluide dans l'échantillon, un séparateur diphasique (9) pour recueillir les fluides issus de l'échantillon suite à l'injection du deuxième fluide, et des moyens (12, 13) d'évacuation du premier fluide en excès, **caractérisé en ce qu'**il comporte des moyens de pesage (10) du séparateur diphasique (9), produisant des signaux indicatifs des variations de la masse du séparateur diphasique (9), des moyens automatiques (14-15) de traitement des signaux produits par les moyens de pesage (10) pour déterminer par discrimination les masses respectives du premier fluide et du deuxième fluide recueillis dans le séparateur diphasique, ainsi que les variations continues de saturation de l'échantillon.

5. Dispositif selon la revendication 4, **caractérisé en ce que** le séparateur diphasique (9) comporte une cellule pourvue d'une entrée pour une canalisation (11) de collecte des fluides évacués de l'échantillon, et d'une sortie pour la connexion d'une canalisation (12) permettant l'évacuation du premier fluide en excès.

6. Dispositif selon l'une des revendications 4 ou 5, **caractérisé en ce que** les moyens de traitement des signaux comportent un processeur (14-15) programmé pour déterminer les variations de saturation à partir des variations de la masse (M) du séparateur diphasique et des masses spécifiques respectives (Rw, Ro) du premier et du deuxième fluide.

**Patentansprüche**

1. Verfahren zur Messung der Änderungen der globalen Sättigung einer eine gewisse Porosität aufweisenden Probe, die man anfänglich mit einem ersten Fluid gesättigt hat, wenn man durch sie ein zweites inkompressibles Fluid bekannter spezifischer Masse unterschiedlich zu der des ersten Fluids und mit diesem nicht mischbar injiziert, wobei man die aus der Probe stammenden Fluide anschließend an die Injektion des zweiten Fluids in einem Zweiphasenseparator (9) sammelt, **dadurch gekennzeichnet, dass** es die anfängliche vollständige Füllung des Phasenseparators mit dem ersten Fluid, die kontinuierliche Messung der Massenveränderungen des Phasenseparators bei progressivem Abzug des im Überschuss vorhandenen ersten Fluids, wodurch eine automatische Diskriminierung der Beiträge beider Phasen zu dieser Massenveränderung ermöglicht und die Berechnung der Veränderungen der globalen Sättigung der Probe als Funktion der Zeit, ausgehend von diesen Massenveränderungen, umfasst.

2. Verfahren nach Anspruch 1, **dadurch gekennzeichnet, dass** man die Probe anfänglich mit Wasser sättigt und man dieses Wasser aus der Probe durch eine Ölinjektion verdrängt.

3. Verfahren nach Anspruch 1, **dadurch gekennzeichnet, dass** man die Probe anfänglich mit Öl sättigt und man dieses Öl aus der Probe durch eine Wasserinjektion verdrängt.

4. Vorrichtung zur kontinuierlichen Messung der Änderungen der globalen Sättigung einer eine gewisse Porosität aufweisenden Probe, die man anfänglich mit einem ersten Fluid gesättigt hat, wenn man durch sie ein zweites mit dem ersten Fluid nicht mischbars Fluid von einer spezifischen Masse unterschiedlich zu der des ersten Fluids injiziert, umfassend: Mittel (3, 5-8) zur Injizierung des zweiten Fluids in die Probe, einen Zweiphasenseparator (9) zum Sammeln der aus der Probe stammenden Fluide anschließend an die Injektion des zweiten Fluids und Mittel (12, 13) zum Abzug des ersten im Überschuss vorhandenen Fluids, **dadurch gekennzeichnet, dass** sie Mittel (10) zum Wiegen des Zweiphasenseparators (9), welche Signale, die indikativ für die Massenveränderungen des Zweiphasenseparators (9) sind, erzeugen und Mittel (14-15) zur automatischen Verarbeitung der durch die Wiegemittel (10) erzeugten Signale, umfasst, um durch Diskriminierung die jeweiligen Massen des ersten Fluids und zweiten Fluids, die im Zweiphasenseparator gesammelt wurden sowie die kontinuierlichen Sättigungsveränderungen der Probe, zu bestimmen.

5. Vorrichtung nach Anspruch 4, **dadurch gekennzeichnet, dass** der Zweiphasenseparator (9) eine Zelle umfasst, die mit einem Eingang für einen Sammelkanal (11) für die aus der Probe abgezogenen Fluide und mit einem Ausgang zum Anschluss eines Kanals (12), der den Abzug des im Überschuss vorhandenen ersten Fluids ermöglicht, versehen ist.

**6.** Vorrichtung nach einem der Ansprüche 4 oder 5, **dadurch gekennzeichnet, dass** die Mittel zur Verarbeitung der Signale einen Prozessor (14-15) umfassen, der so programmiert ist, dass er die Sättigungsveränderungen, ausgehend von den Veränderungen der Masse (M) des Zweiphasenseparators und der jeweiligen spezifischen Massen (Rw, Ro) des ersten und des zweiten Fluids, bestimmt.

**Claims**

**1.** Method for measuring the variations in the global saturation of a sample exhibiting a certain porosity and initially saturated with a first fluid when a second incompressible fluid of known specific gravity different to that of the first fluid and not miscible with it is injected through the sample, wherein the fluids coming from the sample following injection of the second fluid are collected in a two-phase separator (9), **characterised in that** it comprises complete initial filling of the phase separator with the first fluid, continuous measurement of the variations in the mass of the phase separator with progressive discharge of the surplus first fluid, allowing automatic discrimination of the contributions of the two phases to this variation in mass, and calculation of the variations in the global saturation of the sample as a function of the time from said variations in mass.

**2.** Method according to claim 1, **characterised in that** the sample is initially saturated with water and this water is driven out of the sample by injection of oil.

**3.** Method according to claim 1, **characterised in that** the sample is initially saturated with oil and this oil is driven out of the sample by injection of water.

**4.** Device for continuously measuring the variations in the global saturation of a sample exhibiting a certain porosity and initially saturated with a first fluid when a second fluid not miscible with the first fluid and of different specific gravity to that of the first fluid is injected through the sample, comprising means (3, 5-8) for injecting the second fluid into the sample, a two-phase separator (9) for collecting the fluids coming from the sample following injection of the second fluid, and means (12, 13) for discharging the surplus first fluid, **characterised in that** it comprises means (10) for weighing the two-phase separator (9), producing signals indicative of the variations in the mass of the two-phase separator (9), means (14-15) for automatic processing of the signals produced by the weighing means (10) to determine by discrimination the respective masses of the first fluid and the second fluid collected in the two-phase separator, and the continuous variations in the saturation of the sample.

**5.** Device according to claim 4, **characterised in that** the two-phase separator (9) comprises a cell provided with an inlet for a pipe (11) for collecting the fluids discharged from the sample, and an outlet for connection of a pipe (12) allowing discharge of the surplus first fluid.

**6.** Device according to one of claims 4 or 5, **characterised in that** the means for processing the signals comprise a processor (14-15) programmed to determine the variations in the saturation from the variations in the mass (M) of the two-phase separator and the respective specific gravities (Rw, Ro) of the first and the second fluid.